# EUROPEAN PATENT SPECIFICATION

(11) **EP 1 611 898 B1**
(45) Date of publication and mention of the grant of the patent: **01.10.2008**
(21) Application number: 04721551.2
(22) Date of filing: 18.03.2004
(51) Int. Cl.: A61K 38/06, A61P 39/06

(54) **COMPOSITION FOR THE PREVENTION AND TREATMENT OF CELLULITIS**
ZUSAMMENSETZUNG ZUR VERMEIDUNG UND BEHANDLUNG VON CELLULITIS
COMPOSITION PERMETTANT DE PREVENIR ET DE TRAITER LA CELLULITE

(30) Priority: 01.04.2003 ES 200300770
(43) Date of publication of application: 04.01.2006
(73) Proprietor: LIPOTEC, S.A., 08850 Gava (Barcelona) (ES)
(72) Inventor: Garcia Anton, José Maria, 08017 Barcelona (ES); Cebrian Puche, Juan, 08018 Barcelona (ES); Germa Valles, Alejandro José, 08191 Barcelona (ES); Passerini, Elena, Barcelona (ES)
(74) Representative: Davila Baz, Angel
(86) International application number: PCT/ES2004/000126
(87) International publication number: WO 2004/087191

(56) References cited:
- WO-A1-97/17056
- DE-A- 4 244 415
- DE-A- 4 244 418
- FR-A- 2 671 487
- WILLIAMS M.V. ET AL.: 'Glycyl-L-histidyl-L-lysine, a growth promoting factor for human cells' CYTOBIOS vol. 27, 1980, pages 19 - 25, XP001223544
- SAINIO E.L. ET AL: 'Ingredients and safety of cellulite creams.' EUROPEAN JOURNAL OF DERMATOLOGY : EJD DEC 2000 vol. 10, no. 8, pages 596 - 603 ISSN: 1167-1122

## Description

### FIELD OF THE INVENTION

The present invention relates to a composition for topical application for preventing and treating cellulite, comprising classical anticellulite extracts with a lipolytic and/or venotonic effect and the tripeptide GHK (glycyl-histidyl-lysine).

### STATE OF THE ART

From the medical point of view, cellulite is a condition that is due to a reduced microcirculation, damaging the fatty tissue under the skin.

Aesthetically cellulite is a problem consisting of the visual presentation of weakened and damaged fatty tissue and connective fibrous tissue in the form of bumps and dimples, commonly known as "orange skin".

Cellulite is usually located on the hips and buttocks, especially in women. It affects about 80 to 95% of women regardless of whether or not they are overweight or thin, while it only affects 5% of men due to the differences of fat levels and the hormonal system. It is a complex process having numerous causes of a different nature: diet, vascular, endocrine, way of life...

Women have subcutaneous fat that is structured in small chambers, separated by vertical walls of tissue. The entire structure is irrigated by a complex vascular system of arterioles and capillaries, responsible for circulation and draining.

Poor functioning of this microcirculation system results in the loss of fluid to the surrounding tissue. This loss of fluids to interstitial spaces also affects adipocytes that begin to produce excessive triglycerides and increase in size, remaining trapped in the connective structure. This tissue congestion causes swelling, prevents the transport of nutrients such as oxygen to the tissue, and prevents the draining of toxins. A negative cycle begins when increased adipocytes press against the microcirculation system, causing a greater loss of fluids and greater increase of adipocytes.

The connective tissue, including collagen and elastin, becomes increasingly damaged until eventually causing the formation of stretch marks.

The cosmetic result is an irregular distribution of damaged tissue and fat, which modifies the appearance of the skin, causing known "orange skin" dimples.

Cosmetic cellulite treatment must act in two different ways: it must act on the fat (lipolytic effect) and on circulation (venotonic effect). The lipolytic effect is achieved by means of the administration of substances that accelerate fatty acid decomposition within cells, for example by means of stimulating enzymes involved in said decomposition. In this manner the size of the adipocytes is reduced and optical smoothening of the treated skin is obtained. Nevertheless this acceleration of fatty acid decomposition results in the formation of numerous compounds within the cells, some of which may be harmful or toxic for cells. Free radicals, which are involved in cellular aging, stand out among these compounds. An important group of free radicals are the reactive carbonyl species (RCS), formed in oxidative biological processes. Unsaturated aldehydes stand out among these species. Polyunsaturated fatty acid decomposition in cells forms harmful and toxic unsaturated α,β-aldehydes.

In natural cell protection mechanisms the RCS are quenched by certain quenching substances present in the cells for the purpose of preventing their toxic or harmful effects on the cell. Nevertheless this "quenching" by natural RCS mechanisms in cells is insufficient when fatty acid decomposition is accelerated, for example by means of the application of agents with a lipolytic effect.

Therefore there is a need to aid in this RCS "quenching" in cells in which agents with a lipolytic effect are applied to prevent their premature aging.

### DESCRIPTION OF THE INVENTION

DE 42 44 415 discloses the tripeptide GHK (glycyl - histidyl - lysine) and its use in medicine and cosmetics. The peptide is reported to have collagen synthesis stimulating and radical scaranging effects which render it useful as anti-ageing-factor.

It has now surprisingly been found that the tripeptide GHK (glycyl-histidyl-lysine) of formula 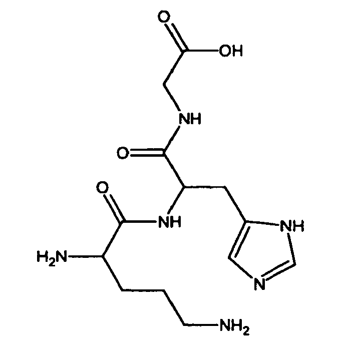 is a good quenching agent of said aldehydes and of other RCS (reactive carbonyle species) present in cells. This activity will be explained below in the present invention, specifically in the examples. This RCS quenching activity may be useful in preparing cosmetic preparations for treating and preventing cellulite, aiding in preventing cellular aging and improving the optical presence of the skin.

Therefore according to a first aspect the present invention relates to a composition for topical, cosmetic or pharmaceutical application for treating and preventing cellulite which reduces the harmful or toxic effects of RCS, comprising classical anticellulite extracts with a lipolytic and/or venotonic effect, and the tripeptide GHK (glycyl-histidyl-lysine) as quenching agent of RCS (reactive carbonyle species).

The composition specifically comprises:
- Caffeine
- Dry butcher's broom extract (Ruscus Aculeatus)
- Triethanolamine hydroiodide
- Ivy extract (Hedera helix)
- L-carnitine
- Amorphous escin
- GHK (glycyl-histidyl-lysine)
- Cosmetically or pharmaceutically acceptable carriers and excipients
- Water.

Caffeine is known for its lipolytic effect: it blocks enzymes responsible for destroying AMPc, which is involved in cleaving triglycerides. It also has vasodilating properties, increasing the blood flow. It therefore contributes to the lipolytic effect and the venotonic effect of the composition.

Dry butcher's broom extract mainly acts on microcirculation, reducing capillary permeability due to its content in a flavonoid called "rutin".

Iodide compounds such as triethanolamine hydroiodide have effective lipolytic properties by means of lipase stimulation.

Ivy extract contains hederin, an active saponin responsible for protecting blood vessels and decreasing permeability. Ivy aids in reabsorbing edemas present in the initial stage of cellulite.

L-carnitine is known for improving triglyceride levels and for accelerating their decomposition.

Escin is a venotonic ingredient with effects on edema reduction.

For its part GHK, as previously explained, is a good quenching agent of aldehydes and other RCS present in cells due to fatty acid decomposition, and it aids in preventing cellular aging and improves the optical presence of the skin.

Carriers and excipients known in the cosmetic or pharmaceutical industry can be used as carriers and excipients. Among them are lecithin, EDTA, urea imidazolidinyl, phenoxyethanol, methylparaben, butylparaben, propylparaben, isobutylparaben, ethylparaben, carrageenans, glycerin, and xanthan gum.

The composition preferably comprises the active ingredients in the following percentages by weight:

| **INGREDIENT** | % |
|---|---|
| Triethanolamine hydroiodide | 0.1 - 10 |
| Caffeine | 0.1 - 10 |
| L-carnitine | 0.1 - 10 |
| Dry butcher's broom extract | 0.1 - 10 |
| Amorphous escin | 0.1 - 10 |
| Ivy extract | 0.1 - 10 |
| GHK (Glycyl-Histidyl-Lysine) | 0.005 - 10 |

The excipients and carriers are preferably incorporated in the following percentages by weight:

| **INGREDIENT** | % |
|---|---|
| Lecithin | 0.1-10 |
| EDTA | 0.1 - 10 |
| Urea imidazolidinyl | 0.1 - 10 |
| Phenoxyethanol, Methylparaben, Butylparaben, Propylparaben, Isobutylparaben, Ethylparaben | 0.1 - 10 |
| Carrageenans | 0.005 - 10 |
| Glycerin | 0.1 - 20 |
| Xanthan gum | 0.1 - 10 |
| Water | 1 - 99 (up to 100%) |

Therefore according to a preferred embodiment the relates to a cosmetic or pharmaceutical composition, the ingredients of which are comprised within the following percentages by weight:

| | |
|---|---|
| • Triethanolamine hydroiodide | 0.1 - 10 |
| • Caffeine | 0.1 - 10 |
| • L-carnitine | 0.1 - 10 |
| • Dry butcher's broom extract | 0.1 - 10 |
| • Amorphous escin | 0.1 - 10 |
| • Ivy extract | 0.1 - 10 |
| • GHK (Glycyl-Histidyl-Lysine) | 0.005 - 10 |
| • Cosmetically or pharmaceutically acceptable carriers and excipients, water: up to 100%. | |

According to another preferred embodiment, the cosmetically or pharmaceutically acceptable carriers and excipients are chosen from the group consisting of lecithin, EDTA, urea imidazolidinyl, phenoxyethanol, methylparaben, butylparaben, propylparaben, isobutylparaben, ethylparaben, carrageenans, glycerin and xanthan gum.

According to an additional preferred embodiment the carriers and excipients and water are contained in the following percentages by weight:

| | |
|---|---|
| • Lecithin | 0.1 - 10 |
| • EDTA | 0.1-10 |
| • Urea imidazolidinyl | 0.1-10 |
| • Phenoxyethanol, Methylparaben, Butylparaben, Propylparaben, Isobutylparaben, Ethylparaben | 0.1 - 10 |
| • Carrageenans | 0.005 - 10 |
| • Glycerin | 0.1 - 20 |
| • Xanthan gum | 0.1 - 10 |
| • Water | 1 - 99 (up to 100%) |

According to a second aspect, the invention relates to the use of the glycyl-histidyl-lysine tripeptide as quenching agent of RCS in the preparation of a cosmetic or pharmaceutical composition, for preventing and treating cellulite, comprising ingredients with lipolytic and venotonic effects.

According to a preferred embodiment, the present invention relates to the use of the tripeptide glycyl-histidyl-lysine as quenching agent of RCS in the preparation of a cosmetic or pharmaceutical composition, comprising ingredients with lipolytic and venotonic effects, chosen from the group comprising caffeine, dry butcher's broom extract, triethanolamine hydroiodide, ivy extract (Hedera helix), L-carnitine and amorphous escin.

The invention will be explained below by means of a series of examples with a character that does not limit the invention:

### EXAMPLES

### Example 1

A composition for preventing and treating cellulite has been prepared according to the present invention by mixing the following ingredients in the indicated amounts (percentages by weight) in Table 1:

**Table 1**

| **INGREDIENT** | % |
|---|---|
| Triethanolamine hydroiodide | 1.5 |
| Caffeine | 2 |
| L-carnitine | 5 |
| Dry butcher's broom extract | 4 |
| Amorphous escin | 4 |
| Ivy extract | 2 |
| Lecithin | 2 |
| EDTA | 0.1 |
| Urea imidazolidinyl | 0.1 |
| Phenoxyethanol, Methylparaben, Butylparaben, Propylparaben, Isobutylparaben, Ethylparaben | 0.5 |
| Carrageenans | 1 |
| Glycerin | 3 |
| Xanthan gum | 0.1 |
| GHK (Glycyl-Histidyl-Lysine) | 0.5 |
| Water | q.s.f. 100 |

### Example 2

The composition for preventing and treating cellulite obtained in Example 1 was used to perform a series of experiments on human adipocytes to determine their activity according to the concentration. Pre-adipocytes obtained from a sample from a white adipose tissue biopsy were seed and cultivated in a medium at a ratio of 1:1 by volume in DMEM (Dulbecco's Modified Eagle's Medium) and Ham Nutrient Mixture F12. Preadipocyte to adipocyte differentiation was induced in a 3-day culture with the same medium mentioned above, supplementing it with 3% FCS (fetal calf serum), 10 µg/ml of insulin, 33 µM of D-biotin, 17 µM of Na pantothenate, 1 µM of dexamethasone, 0.5 mM of IBMX (3-isobutyl-1-methyl-xanthine) and 1 µM of rosiglitazone. This mixture induces a heavy accumulation of fat inside the drops of fat contained in the pre-adipocytes. For the experiments the cultures included the composition of Example 1 at concentrations of 0, 0.1, 1, 10 and 100 µg/ml, in the presence of 1 µM of dexamethasone for 8 days. The lipid drops were viewed by phase contrast microscopy and quantified by means of image analysis. The number of adipocytes per area unit (statistical mean ± standard deviation), among other factors, were quantified during image analysis. The results are shown in the following Graph 1:

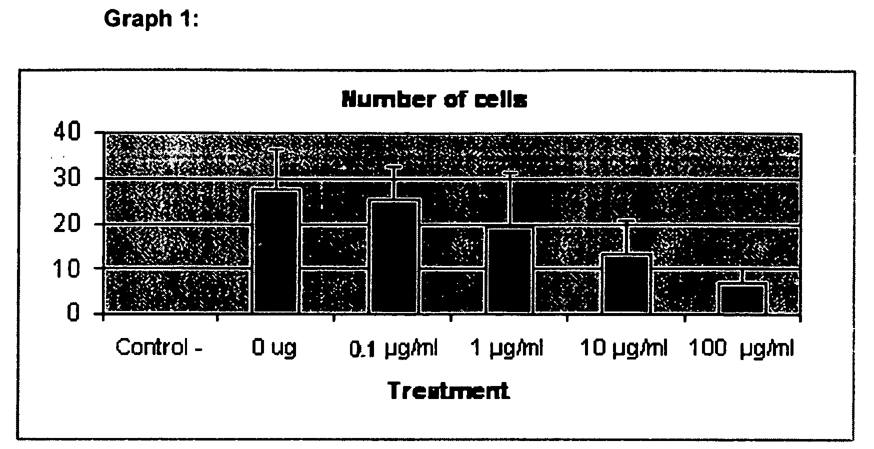

### Example 3

Experiments have been carried out to determine tripeptide GHK activity as a quenching agent of the aldehyde HNE in cells according to the molar ratio of aldehyde with respect to tripeptide GHK. The results are indicated in Graph 2.

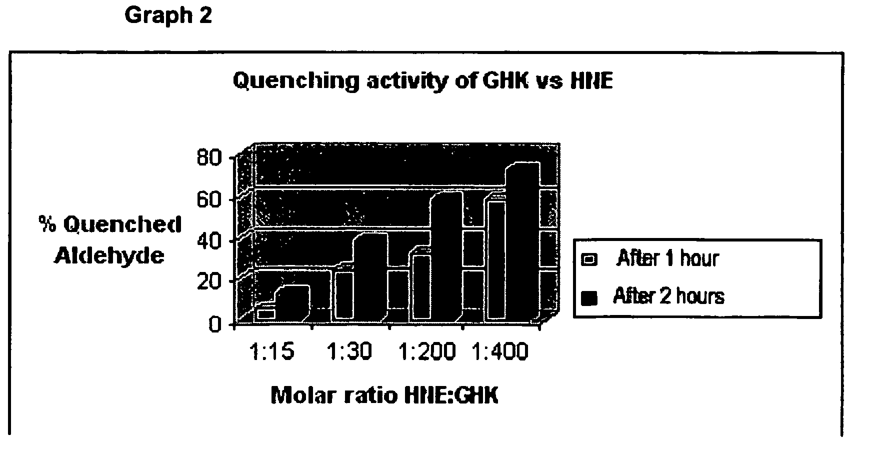

As can be observed, the quenching activity of tripeptide GHK with respect to HNE is high and increases with the molar ratio of GHK with respect to aldehyde, as well as over time.

### Example 4

Experiments have been performed to determine the active of tripeptide GHK as a quenching agent of the aldehyde acrolein in cells according to the molar ratio of aldehyde with respect to tripeptide GHK. The results are indicated in Graph 3.

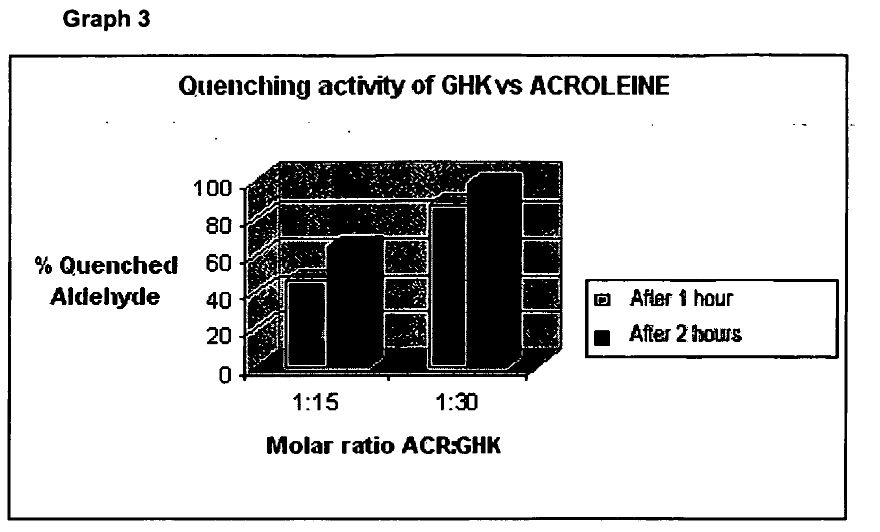

As can be observed the quenching activity of tripeptide GHK with respect to acrolein is very high and increases with the molar ratio of GHK with respect to aldehyde, as well as over time.

## Claims

1. A composition for topical application for preventing and treating cellulite which reduces the harmful or toxic effects of RCS, **characterized in that** it comprises the following ingredients:
• ingredients with lipolytic and venotonic effects;
• GHK (glycyl-histidyl-lysine) as quenching agent of RCS;
• Cosmetically or pharmaceutically acceptable carriers and excipients;
• Water.

2. A composition according to claim 1, **characterized in that** ingredients with lipolytic and venotonic effects are:
• Caffeine;
• Dry butcher's broom extract (Ruscus Aculeatus);
• Triethanolamine hydroiodide;
• Ivy extract (Hedera helix);
• L-carnitine;
• Amorphous escin.

3. A composition according to claim 2, **characterized in that** the ingredients are comprised within the following percentages by weight:
| | |
|---|---|
| • Triethanolamine hydroiodide | 0.1-10 |
| • Caffeine | 0.1 - 10 |
| • L-carnitine | 0.1 -10 |
| • Dry butcher's broom extract | 0.1 - 10 |
| • Amorphous escin | 0.1-10 |
| • Ivy extract | 0.1 - 10 |
| • GHK (Glycyl-Histidyl-Lysine) | 0.005 - 10 |
| • Carriers and excipients, water: | up to 100%. |

4. A composition according to any of claims 1 to 3, **characterized in that** the carriers and excipients are chosen from the group consisting of lecithin, EDTA, urea imidazolidinyl, phenoxyethanol, methylparaben, butylparaben, propylparaben, isobutylparaben, ethylparaben, carrageenans, glycerin and xanthan gum.

5. A composition according to claim 4, **characterized in that** the carriers and excipients and water are contained in the following percentages by weight:
| | |
|---|---|
| • Lecithin | 0.1 - 10 |
| • EDTA | 0.1-10 |
| • Urea imidazolidinyl | 0.1 - 10 |
| • Phenoxyethanol, methylparaben, butylparaben, propylparaben, isobutylparaben, ethylparaben | 0.1 - 10 |
| • Carrageenans | 0.005 - 10 |
| • Glycerin | 0.1 - 20 |
| • Xanthan gum | 0.1 - 10 |
| • Water | 1 - 99 (up to 100%) |

6. Use of the tripeptide glycyl-histidyl-lysineas quenching agent of RCS in preparing a composition for preventing and treating cellulite comprising ingredients with lipolytic and venotonic effects.

7. Use according to claim 6, **characterized in that** the ingredients with lipolytic and venotonic effects are chosen from the group comprising caffeine, dry butcher's broom extract, triethanolamine hydroiodide, ivy extract (Hedera helix), L-carnitine and amorphous escin.

## Patentansprüche

1. Zusammensetzung zur topischen Anwendung, welche zur Vorbeugung und Behandlung von Cellulite dient und welche die schädlichen oder toxischen Wirkungen von RCS vermindert, **dadurch gekennzeichnet, dass** sie folgende Bestandteile umfasst:
- Bestandteile mit lipolytischen und venenstärkenden Wirkungen;
- GHK (Glycyl-Histidyl-Lysin) als Mittel zum Abfangen von RCS;
- Trägerstoffe und pharmazeutisch unwirksame Bestandteile, die kosmetisch oder pharmazeutisch unbedenklich sind;
- Wasser.

2. Zusammensetzung nach Anspruch 1, **dadurch gekennzeichnet, dass** es sich bei den Bestandteilen mit lipolytischen und venenstärkenden Wirkungen um die folgenden handelt:
- Coffein;
- Extrakt aus getrocknetem stechenden Mäusedorn (Ruscus Aculeatus);
- Triethanolamin-Hydroiodid;
- Efeu-Extrakt (Hedera helix);
- L-Carnithin;
- amorphes Aescin.

3. Zusammensetzung nach Anspruch 2, **dadurch gekennzeichnet, dass** die Bestandteile zu den folgenden gewichtsmäßigen Prozentsätzen darin enthalten sind:
| | |
|---|---|
| - Triethanolamin-Hydroiodid | 0,1 - 10 |
| - Coffein | 0,1 - 10 |
| - L-Carnithin | 0,1 - 10 |
| - Extrakt aus getrocknetem stechenden Mäusedorn | 0,1 - 10 |
| - amorphes Aescin | 0,1 - 10 |
| - Efeu-Extrakt | 0,1 - 10 |
| - GHK (Glycyl-Histidyl-Lysin) | 0,005 - 10 |
| - Trägerstoffe und pharmazeutisch unwirksame Bunwirksame estandteile, Wasser: | ad 100 %. |

4. Zusammensetzung nach einem beliebigen der Ansprüche 1 bis 3, **dadurch gekennzeichnet, dass** die Trägerstoffe und pharmazeutisch unwirksamen Bestandteile aus der Gruppe gewählt sind, die aus Lecithin, EDTA, Imidazolidinyl-Harnstoff, Phenoxyethanol, Methylparaben, Butylparaben, Propylparaben, Isobutylparaben, Ethylparaben, Carrageenen, Glycerin und Xanthan besteht.

5. Zusammensetzung nach Anspruch 4, **dadurch gekennzeichnet, dass** die Trägerstoffe und pharmazeutisch unwirksamen Bestandteile zu den folgenden gewichtsmäßigen Prozentsätzen enthalten sind:
| | |
|---|---|
| - Lecithin | 0,1 - 10 |
| - EDTA | 0,1 - 10 |
| - Imidazolidinyl-Harnstoff | 0,1 - 10 |
| - Phenoxyethanol, Methylparaben, Butylparaben, Propylparaben, Isobutylparaben, Ethylparaben | 0,1 - 10 |
| - Carrageene | 0,005 - 10 |
| - Glycerin | 0,1 - 20 |
| - Xanthan | 0,1 - 10 |
| - Wasser | 1 bis 99 (ad 100 %) |

6. Verwendung des Tripeptids Glycyl-Histidyl-Lysin als Mittel zum Abfangen von RCS beim Herstellen einer Zusammensetzung zur Vorbeugung und Behandlung von Cellulite, welche Bestandteile mit lipolytischen und venenstärkenden Wirkungen umfasst.

7. Verwendung nach Anspruch 6, **dadurch gekennzeichnet, dass** die Bestandteile mit lipolytischen und venenstärkenden Wirkungen aus der Gruppe gewählt sind, die Coffein, Extrakt aus getrocknetem stechenden Mäusedorn, Triethanolamin-Hydroiodid, Efeu-Extrakt (Hedera helix), L-Carnithin und amorphes Aescin umfasst.

## Revendications

1. Composition pour application topique destinée à prévenir et à traiter la cellulite, qui réduit les effets néfastes ou toxiques des RCS, **caractérisée en ce qu'**elle comprend les ingrédients suivants :
• ingrédients ayant des effets lipolytiques et vénotoniques ;
• GHK (glycyl-histidyl-lysine) comme agent modérateur des RCS ;
• supports et excipients cosmétiquement ou pharmaceutiquement acceptables ;
• eau.

2. Composition selon la revendication 1, **caractérisée en ce que** les ingrédients ayant des effets lipolytiques et vénotoniques sont :
• la caféine ;
• l'extrait de fragon épineux sec (Ruscus Aculeatus) ;
• l'hydroiodure de triéthanolamine ;
• l'extrait de lierre (Hedera helix) ;
• la L-carnitine ;
• l'escine amorphe.

3. Composition selon la revendication 2, **caractérisée en ce que** les ingrédients sont présents dans les pourcentages en poids suivants :
| | |
|---|---|
| • hydroiodure de triéthanolamine | 0,1 - 10 |
| • caféine | 0,1 - 10 |
| • L-carnitine | 0,1 - 10 |
| • extrait de fragon épineux sec | 0,1 - 10 |
| • escine amorphe | 0,1 - 10 |
| • extrait de lierre | 0,1 - 10 |
| • GHK (glycyl-histidyl-lysine) | 0,005 - 10 |
| • supports et excipients, eau : jusqu'à 100%. | |

4. Composition selon l'une quelconque des revendications 1 à 3, **caractérisée en ce que** les supports et excipients sont choisis dans le groupe comprenant la lécithine, l'EDTA, l'imidazolidinylurée, le phénoxyéthanol, le méthylparabène, le butylparabène, le propylparabène, le sobutylparabène, l'éthylparabène, les carraghéinines, la glycérine et la gomme xanthique.

5. Composition selon la revendication 4, **caractérisée en ce que** les supports et excipients et l'eau sont contenus dans les pourcentages suivants en poids :
| | |
|---|---|
| • lécithine | 0,1 - 10 |
| • EDTA | 0,1 - 10 |
| • imidazolidinylurée | 0,1 - 10 |
| • phénoxyéthanol méthylparabène, butylparabène, propylparabène, isobutylparabène, éthylparabène, | 0,1 - 10 |
| • carraghéinines | 0,005 - 10 |
| • glycérine | 0,1 - 20 |
| • gomme xanthique | 0,1 - 10 |
| • eau | 1 - 99 |
| | (jusqu'à 100%) |

6. Utilisation du glycyl-histidyl-lysine de tripeptide comme agent modérateur des RCS dans la préparation d'une composition destinée à prévenir et à traiter la cellulite comprenant des ingrédients ayant des effets lipolytiques et vénotoniques.

7. Utilisation selon la revendication 6, **caractérisée en ce que** les ingrédients ayant des effets lipolytiques et vénotoniques sont choisis dans le groupe comprenant la caféine, l'extrait de fragon épineux sec, l'hydroiodure de triéthanolamine, l'extrait de lierre (hedera helix), la L-carnitine et l'escine amorphe.
